Europäisches Patentamt

(19) European Patent Office

Office européen des brevets

(11) Veröffentlichungsnummer: **0 144 688**
**B1**

(12) # EUROPÄISCHE PATENTSCHRIFT

(45) Veröffentlichungstag der Patentschrift:
**14.09.88**

(51) Int. Cl.⁴: **A 61 K 7/06**

(21) Anmeldenummer: **84112917.4**

(22) Anmeldetag: **26.10.84**

(54) **Haarkurmittel und Verfahren zur Haarbehandlung.**

(30) Priorität: **08.11.83 DE 3340350**

(43) Veröffentlichungstag der Anmeldung:
**19.06.85 Patentblatt 85/25**

(45) Bekanntmachung des Hinweises auf die Patenterteilung:
**14.09.88 Patentblatt 88/37**

(84) Benannte Vertragsstaaten:
**AT BE CH DE FR GB IT LI LU NL SE**

(56) Entgegenhaltungen:
**DE - A - 2 641 748**
**DE - A - 2 822 358**
**GB - A - 1 555 796**
**US - A - 3 098 795**

**Die Akte enthält technische Angaben, die nach dem Eingang der Anmeldung eingereicht wurden und die nicht in dieser Patentschrift enthalten sind.**

(73) Patentinhaber: **Wella Aktiengesellschaft, Berliner Allee 65, D-6100 Darmstadt (DE)**

(72) Erfinder: **Gross, Paul, Forstweg 54, D-6100 Darmstadt (DE)**
Erfinder: **Keller, Walter, Im Höhlchen 2a, D-6105 Ober-Ramstadt (DE)**

ACTORUM AG

## Beschreibung

Gegenstand der Erfindung sind Haarkurmittel, die Cetylalkoholmilchsäureester, nachfolgend Cetyllactat genannt, und mindestens eine quaternäre monomere Ammoniumverbindung in alkoholischer Lösung enthalten sowie Haarbehandlungsverfahren unter Verwendung dieser Kurmittel.

Durch öfteres Bleichen, Dauerwellen und Färben, aber auch durch häufiges Waschen der Haare mit entfettenden Tensiden sowie durch intensive Einwirkung von Sonnenlicht, kommt es zu einer Schädigung der Haarstruktur. Das Haar wird spröde, und es verliert seinen Glanz. Weiterhin lädt sich das Haar beim Kämmen elektrostatisch auf, und die aufgerauhte Haaroberfläche verursacht Verfilzungen sowie Verknotungen des Haares. Hierdurch wird das Kämmen sehr erschwert. Haarkurmittel mit einer kämmbarkeitsverbessernden und pflegenden Wirkung haben daher eine erhebliche Bedeutung erlangt.

Haarkurmittel dienen der Verbesserung der Haarkondition und sind üblicherweise Öl-in-Wasser-Emulsionen, die als Gerüstbestandteile ölige, halbfeste oder feste Substanzen, wie beispielsweise Paraffinöl, Vaseline, Wollfett, Wollfettalkohole, Fettsäureester und Kohlenwasserstoffwachse enthalten. Als Emulgatoren für solche Emulsionen werden normalerweise quaternäre Ammoniumverbindungen wie oxethylierte Alkylammoniumphosphate, Alkyltrimethylammoniumchloride, Dialkyldimethylammoniumchloride, Alkyldimethylbenzylammoniumchloride und Alkylpyridiniumchloride, allein oder in Kombination mit nichtionogenen Emulgatoren, verwendet. Auch wässrige Lösungen und wässrige Gele mit einem Gehalt an quaternären Ammoniumverbindungen finden häufig als Haarkurmittel Anwendung. Derartige Mittel werden beispielsweise häufig in Form einer klaren Haarpflegespülung oder auch in Emulsionsform als sogenannte Creme-Rinses nach der Haarwäsche im noch nassen Haar verteilt, einige Minuten einwirken gelassen und dann mit Wasser ausgespült.

Mit Haarkurmitteln auf der Basis von monomeren quaternären Ammoniumverbindungen kann die Nasskämmbarkeit und der Griff des Haares in gewissem Umfang verbessert werden.

Ferner ist aus der DE-A-2 822 358 eine alkoholfreie Cremespülung mit einem Wassergehalt von über 90 Gew.% bekannt, welche 1,75 Gew.% Stearyldimethylbenzylammoniumchlorid und 1,0 Gew.% Cetyllactat enthält. Im Patentanspruch 1 dieser Druckschrift, welcher Haareinleg- und Haarkonditionierungspräparate umfasst, ist zwar angegeben, dass die Lösungsmittelgrundlage III. u.a. auch aus aliphatischen $C_{2-3}$ Alkoholen ausgewählt werden kann, jedoch soll, gemäss Seite 15 Absatz 2 der Beschreibung, bei konditionierenden Spülungen zweckmässig dem Lösungsmittel der Alkohol fehlen. In der DE-A-2 641 748 ist eine alkoholfreie cremige Haarbefeuchungslotion (Lotionbalsam) mit einem Wassergehalt von etwa 83 Gew.% beschrieben, die ferner 2,0 Gew.% Cetyllactat und 0,1 Gew.% eines quaternären Ammoniumgermicidsalzes enthält. Der Gehalt von 0,1 Gew.% des quaternären Ammoniumsalzes ist für eine haarkonditionierende Wirkung zu gering. Die in den vorstehend genannten Dokumenten aufgeführten Haarkurmittel liegen in Form von Öl-in-Wasser-Emulsionen vor. Bedingt durch die zur Erzeugung der Emulsion erforderlichen hydrophoben wachsartigen Bestandteile sowie dem Fehlen von Alkohol, können sie hinsichtlich Nass- und Trockenkämmbarkeit sowie Glanz und Griff des damit behandelten Haares nicht voll befriedigen.

Aus der US-A-3 098 795 sind Mittel zum Frisieren der Haare bekannt, welche in ethanolischer Lösung 5 Gew.% Cetyllactat enthalten und mit einem Treibgas als Aerosolspray versprüht werden sollen. Eine Anregung, diesem Mittel eine quaternäre Ammoniumverbindung hinzuzufügen, ist dort nicht gegeben.

Es ist auch bekannt, in Mitteln für die Behandlung von Haaren kationaktive Polymere, insbesondere Polymere, die quaternäre Ammoniumgruppen aufweisen, als Konditionierungsmittel einzusetzen. Auf Grund einer Wechselwirkung zwischen ihren Ammoniumgruppen und den anionischen Gruppen des Haares besitzen die kationaktiven Polymere eine grosse Affinität zur Keratinfaser.

Es wurde festgestellt, dass der Einsatz derartiger kationaktiver Polymere in solchen kosmetischen Mitteln zahlreiche Vorteile ergibt; die Entwirrung der Haare sowie deren Behandlung wird erleichtert, und weiterhin wird dem Haar Sprungkraft und Glanzwirkung verliehen. Durch die Affinität zum Keratin neigen diese Polymere jedoch bei wiederholter Anwendung zur Ansammlung auf den Haaren, so dass diese schwerer werden, was im Endeffekt unerwünscht ist.

Wie sich in der Praxis gezeigt hat, können die Haarkurmittel nach dem Stand der Technik hinsichtlich ihrer haarkonditionierenden Wirkung, insbesondere was den Griff, Glanz sowie die Kämmbarkeit des Haares anbetrifft, noch nicht voll zufriedenstellen.

Demgegenüber wurde nun gefunden, dass Haarkurmittel mit einem Gehalt an Cetylalkoholmilchsäureester und einer monomeren quaternären Ammoniumverbindung, dadurch gekennzeichnet, dass es in Form einer ethanolischen und/oder isopropanolischen Lösung vorliegt und 0 bis 15 Gew.% Wasser, 0,1 bis 40 Gew.% des Cetylalkoholmilchsäureesters und 0,5 bis 30 Gew.% der monomeren quaternären Ammoniumverbindung enthält, eine überraschende und ausgezeichnete Verbesserung der Nass- und Trockenkämmbarkeit des Haares sowie einen verbesserten Griff, insbesondere des geschädigten Haares, bewirken. Darüber hinaus ist ein hervorragender Glanz des Haares festzustellen.

Die beobachtete ausgezeichnete Verbesserung der Kämmbarkeit, des Glanzes und des Griffes ist überraschend und nur synergistisch erklärbar, da Cetyllactat oder quaternäre monomere Ammoniumverbindungen in entsprechender Lösung allein auf das Haar aufgebracht nur eine mässige und unbefriedigende Verbesserung der genannten Kriterien zeigen. Wie Versuche ergeben haben, zeigt, im Gegensatz zu dem erfindungsgemässen Mitteln auf der Basis von Cetyllactat und monomeren quaternären Ammoniumverbindungen, die Kombination von Cetyllactat mit quaternären Polymeren nicht den beobachteten synergistischen Effekt bezüglich der beschriebenen Verbesserung der Kämmbarkeit, des Glanzes und des Griffes des Haares.

Die erfindungsgemässen Haarkurmittel werden, je nach Haarfülle, in einer Menge von etwa 20 bis 50 g entweder in Form der alkoholischen Lösung nach der Haarwäsche in dem noch feuchten Haar verteilt oder vor dem Aufbringen auf das Haar mit Wasser verdünnt, wodurch eine alkoholisch-wässrige Emulsion entsteht und sodann in gleicher Weise aufgebracht. Nach einer Einwirkungszeit von etwa 3–5 Minuten wird das Haar mit Wasser gespült.

Die hier beschriebenen Mittel stellen dünnflüssige oder leicht angedickte alkoholische Lösungen dar, die durch einen Gehalt an der synergistischen Kombination aus Cetyllactat und einer quaternären monomeren Ammoniumverbindung in den angegebenen Mengen gekennzeichnet sind.

In den erfindungsgemässen Haarkurmitteln soll das Cetyllactat in einer Menge von 1 bis 40 Gew.%, vorzugsweise in einer Menge von 8,0 bis 20 Gew.%, enthalten sein. Der Gehalt an der quaternären monomeren Ammoniumverbindung soll 0,5 bis 30 Gew.%, vorzugsweise 1,5 bis 10,0 Gew.%, betragen. Der Isopropylalkohol oder das Ethanol oder Gemische dieser beiden Alkohole sollen in den Mitteln in einer Menge von 30,0 bis 98,5 Gew.%, vorzugsweise 60 bis 95 Gew.%, enthalten sein. Selbstverständlich können die Haarkurmittel darüber hinaus ausserdem noch für Haarbehandlungsmittel übliche kosmetische Bestandteile und Zusätze, wie beispielsweise Parfümöle, Kräuterextrakte, bakterizide oder fungizide Stoffe, Vergällungsmittel, Anfärbefarbstoffe oder direkt auf das Haar aufziehende Farbstoffe, enthalten.

Als übliche Bestandteile von Haarbehandlungsmitteln kommen z.B. Wasser, Aceton, n-Propanol, kationische oder nichtionogene Teniside, oxethylierte Fettalkohole, weiterhin natürliche, modifizierte natürliche oder synthetische Polymere, wie zum Beispiel Chitosan, ferner Verdicker, wie Fettalkohole, Fettsäureester, Cellulosederivate, Paraffine, Isoparaffine, Vaseline und Wollwachs sowie ausserdem Pflegestoffe wie Lanolinderivate, Cholesterin und Pantothensäure, Antioxidantien, Konservierungsmittel und Antischuppenmittel, wie zum Beispiel Salicylsäure, weiterhin Pigmente und Farbstoffe, in Betracht.

Von den bekannten kosmetischen Farbstoffen, die einzeln oder in Mischung vorliegen können, seien beispielsweise die folgenden Klassen erwähnt: Aromatische Nitrofarbstoffe (z.B. 1,4-Diamino-2-nitrobenzol), Azofarbstoffe (z.B. Acid Brown 4), Anthrachinonfarbstoffe (z.B. C.I. Disperse Violet 4) und Triphenylmethanfarbstoffe (z.B. Basic Violet 1), wobei die Farbstoffe dieser Klassen je nach der Art ihrer Substituenten sauren, nichtionogenen oder basischen Charakter haben können. Ihre Gesamtkonzentration beträgt üblicherweise etwa 0,05 bis 2,0 Gew.%.

Wird das erfindungsgemässe Haarkurmittel vor der Anwendung auf dem Haar mit Wasser verdünnt, so kann übliches Leitungswasser verwendet werden. Das zur Verdünnung verwendete Wasser kann aber auch dem erfindungsgemässen Mittel getrennt beigepackt werden und seinerseits übliche haarkosmetische Stoffe, wie beispielsweise Kräuterextrakte, bakterizide oder fungizide Stoffe, Anfärbefarbstoffe oder auf das Haar aufziehende Farbstoffe enthalten, wenn dies von Vorteil ist.

Die erfindungsgemässen Mittel in Form von alkoholischen Lösungen können mit Wasser oder mit der beigepackten wässrigen Lösung bis zu einem Verhältnis von 1 : 10, vorzugsweise im Verhältnis 1 : 2 bis 1 : 4, verdünnt werden.

Die folgenden Beispiele sollen den Gegenstand der Erfindung näher erläutern, ohne ihn darauf zu beschränken.

Beispiele

Beispiel 1:
　10 g Cetyllactat
　　4 g Distearyldimethylammoniumchlorid, 75%-ig (Rest: Wasser)
　86 g Ethanol, rein (94,7 Gew.%-ig)
100 g

Mit je 18 g eines Haarkurmittels gemäss Beispiel 1 wurden 15 Versuchspersonen mit stark geschädigtem Haar nach einer üblichen Haarwäsche behandelt. Um eindeutige Ergebnisse zu erhalten und von Versuchsperson zu Versuchsperson abweichende Haarqualitäten auszuschalten, wurde das Haar in der Mitte gescheitelt und eine Hälfte des Haares mit dem erfindungsgemässen Präparat behandelt, während die andere Hälfte unbehandelt blieb. So konnte die Wirkung des Präparates von einer friseur-fachlichen Expertengruppe sicher beurteilt werden. Eine Bewertung erfolgte nach dem Schema für die zusammengefassten Kriterien Nass- und Trockenkämmbarkeit, Griff und Glanz des Haares wie folgt:

1 = sehr gut
2 = gut
3 = ausreichend
4 = mangelhaft

Das Ergebnis zeigt die nachstehende Tabelle 1.

Tabelle 1

|  | Note 1 | Note 2 | Note 3 | Note 4 |
|---|---|---|---|---|
| Anzahl der Versuchspersonen | 12 | 2 | 1 | — |

Überprüfung des synergistischen Effektes

Zur Überprüfung des synergistischen Effektes wurden die Haare einer weiteren Gruppe von 12 Personen in gleicher Weise wie in Beispiel 1 behandelt, jedoch enthielt das Haarkurmittel kein Cetyllactat. In der Zusammensetzung nach Beispiel 1 wurde dabei das Cetyllactat durch die weitere Zugabe von 10 g Ethanol ersetzt. Das Ergebnis des Tests zeigt die nachstehende Tabelle 2.

Tabelle 2

|  | Note 1 | Note 2 | Note 3 | Note 4 |
|---|---|---|---|---|
| Anzahl der Versuchspersonen | – | 2 | 9 | 1 |

Zur weiteren Überprüfung des synergistischen Effektes wurde eine dritte Gruppe von 12 Personen gemäss Beispiel 1 behandelt, jedoch enthielt das Haarkurmittel diesmal kein Distearyldimethylammoniumchlorid. In der Zusammensetzung nach Beispiel 1 wurde hierzu das Distearyldimethylammoniumchlorid durch die entsprechende Menge Ethanol ersetzt. Das Ergebnis ist in der Tabelle 3 wiedergegeben.

Tabelle 3

|  | Note 1 | Note 2 | Note 3 | Note 4 |
|---|---|---|---|---|
| Anzahl der Versuchspersonen | – | – | 4 | 8 |

Ein Vergleich der Ergebnisse von Tabelle 1, 2 und 3 zeigt deutlich, dass das erfindungsgemässe Mittel gemäss Beispiel 1 eine synergistische Wirkung im Vergleich zu den entsprechenden Mitteln mit einem Gehalt an jeweils nur einer der beiden Komponenten aufweist.

Beispiel 2:
    5 g  Cetyllactat
    2 g  Cetyltrimethylammoniumchlorid, 25%-ige wässrige Lösung
    1 g  Parfümöl
    <u>92 g</u>  Ethynol, rein (94,7 Gew.%-ig)
  100 g

Beispiel 3:
    15 g  Cetyllactat
    3 g  Laurylpyridiniumchlorid (90 bis 94%-ig)
    <u>82 g</u>  Ethanol, rein (94,7 Gew.%-ig)
  100 g

Beispiel 4:
    12,0 g  Cetyllactat
    2,0 g  Tetradecyltrimethylammoniumbromid
    0,1 g  Farbstoff Basic Violet 1 (C.I. Nr. 42 535)
    <u>85,9 g</u>  Ethanol, rein (94,7 Gew.%-ig)
  100,0 g

Beispiel 5:
    20 g  Cetyllactat
    10 g  Tris-(oligooxyethyl)octadecylammoniumphosphat
    <u>70 g</u>  Ethanol, rein (94,7 Gew.%-ig)
  100 g

Beispiel 6:
    11,0 g  Cetyllactat
    6,0 g  Distearyldimethylammoniumchlorid, 75%-ig (Rest:Wasser)
    0,1 g  2,4,4'-Trichlor-2'-hydroxydiphenylether
    <u>82,9 g</u>  Ethynol, rein (94,7 Gew.%-ig)
  100,0 g

Beispiel 7:
    30 g  Cetyllactat
    6 g  Methyl-tri($C_8$–$C_{10}$-alkyl)ammoniumchlorid, 85%-ig
    <u>64 g</u>  Ethanol, rein (94,7 Gew.%-ig)
  100 g

Beispiel 8:
    19 g  Cetyllactat
    6 g  Distearyldimethylammoniumchlorid, 75%-ig (Rest: Wasser)
    <u>75 g</u>  Isopropanol
  100 g

Jeweils 35 g der Haarkurmittel gemäss den Beispielen 2 bis 8 werden im gewaschenen Haar verteilt und nach einer Einwirkungszeit von 3 bis 5 Minuten mit Wasser wieder ausgespült. Als Ergebnis wird ein ausgezeichneter Griff, Glanz sowie Kämmbarkeit des Haares erhalten.

Sämtliche in der Anmeldung angegebenen Prozentzahlen stellen Gewichtsprozente dar.

**Patentansprüche**

1. Haarkurmittel mit einem Gehalt an Cetylalkoholmilchsäureester und einer monomeren quaternären Ammoniumverbindung, dadurch gekennzeichnet, dass es in Form einer ethanolischen und/oder isopropanolischen Lösung vorliegt und 0 bis 15 Gew.% Wasser, 0,1 bis 40 Gew.% des Cetylalkoholmilchsäureesters und 0,5 bis 30 Gew.% der monomeren quaternären Ammoniumverbindung enthält.

2. Mittel nach Anspruch 1, dadurch gekennzeichnet, dass es 8,0 bis 20 Gew.% Cetylalkoholmilchsäureester enthält.

3. Mittel nach Anspruch 1 und 2, dadurch gekennzeichnet, dass es 1,5 bis 10 Gew.% einer monomeren quaternären Ammoniumverbindung enthält.

4. Mittel nach Anspruch 1 bis 3, dadurch gekennzeichnet, dass es 60 bis 95 Gew.% Ethanol und/oder Isopropanol enthält.

5. Mittel nach Anspruch 1 bis 4, dadurch gekennzeichnet, dass die monomere quaternäre Ammoniumverbindung ausgewählt ist aus Distearyldimethylammoniumchlorid, Laurylpyridiniumchlorid, Tetradecyltrimethylammoniumbromid, Tris-(oligooxyethyl)-octadecylammoniumphosphat und Methyltri($C_8$–$C_{10}$-alkyl)ammoniumchlorid und Cetyltrimethylammoniumchlorid.

6. Verfahren zur Haarbehandlung, bei dem man in dem nach der Haarwäsche noch feuchten Haar etwa 20 bis 50 g eines Haarkurmittels verteilt, das Haarkurmittel etwa 3 bis 5 Minuten einwirken lässt und sodann das Haar mit Wasser spült, dadurch gekennzeichnet, dass man ein Haarkurmittel nach Anspruch 1 verwendet.

## Claims

1. Hair treatment agent containing cetyl alcohol lactic acid ester and a monomeric quaternary ammonium compound, characterised in that it takes the form of an ethanolic and/or isopropanolic solution and contains 0 to 15% by weight water, 0.1 to 40% by weight of the cetyl alcohol lactic acid ester and 0.5 to 30% by weight of the monomeric quaternary ammonium compound.

2. Agent according to Claim 1, characterised in that it contains 8.0 to 20% by weight cetyl alcohol lactic acid ester.

3. Agent according to Claim 1 and 2, characterised in that it contains 1.5 to 10% by weight of a monomeric quaternary ammonium compound.

4. Agent according to Claim 1 to 3, characterised in that it contains 60 to 95% weight ethanol and/or isopropanol.

5. Agent according to Claim 1 to 4, characterised in that the monomeric quaternary ammonium compound is chosen from distearyl dimethyl ammonium chloride, laurylpyridinium chloride, tetradecytrimethyl ammonium bromide, tri-(oligooxyethyl)-octadecyl ammonium phosphate and methyltri($C_8$–$C_{10}$-alkyl) ammonium chloride and cetyltrimethyl ammonium chloride.

6. Method of treating hair in which approximately 20 to 50 g of a hair treatment agent are distributed over the hair which is still wet following washing, the hair treatment agent is left for about 3 to 5 minutes to act after which the hair is rinsed with water, characterised in that a hair treatment agent according to Claim 1 is used.

## Revendications

1. Produit pour soins capillaires renfermant d'ester lactique de l'alcool cétylique et d'un composé d'ammonium quaternaire monomère, caractérisé en ce qu'il se présente sous la forme d'une solution dans l'éthanol et/ou l'isopropanol et qu'il renferme 0 à 15% en poids d'eau, 0,1 à 40% en poids d'ester lactique de l'alcool cétylique et 0,5 à 30% en poids du composé d'ammonium quaternaire monomère.

2. Produit selon la revendication 1, caractérisé en ce qu'il renferme 8,0 à 20% en poids d'ester lactique de l'alcool cétylique.

3. Produit selon les revendications 1 et 2, caractérisé en ce qu'il renferme 1,5 à 10% en poids d'un composé d'ammonium quaternaire monomère.

4. Produit selon les revendications 1 à 3, caractérisé en ce qu'il renferme 60 à 95% en poids d'éthano et/ou d'isopropanol.

5. Produit selon les revendications 1 à 4, caractérisé en ce que le composé d'ammonium quaternaire monomère est choisi parmi le chlorure de distéaryldiméthylammonium, le chlorure de lauryl-pyridinium, le bromure de tétradécyltriméthyl-ammonium, le phosphate de tris-(oligo-oxyéthyl)-octadécyl-ammonium et un chlorure de méthyltrialcoylammonium (le groupe acoyle comportant 8 à 10 atomes de carbone), et le chlorure de cétyl-triméthylammonium.

6. Procédé de traitement des cheveux, dans lequel on répartit dans les cheveux encore mouillés après leur lavage environ 20 à 50 g d'un produit pour soins capillaires, on laisse agir le produit pour soins capillaires environ 3 à 5 minutes, puis on rince les cheveux avec de l'eau, caractérisé en ce qu'on utilise un produit pour soins capillaires selon la revendication 1.